Europäisches Patentamt

European Patent Office

Office européen des brevets

(11) Publication number: **0 205 915**
**B1**

(12) **EUROPEAN PATENT SPECIFICATION**

(45) Date of publication of patent specification: **25.07.90**

(51) Int. Cl.⁵: **A 61 M 5/178**, A 61 M 5/32

(21) Application number: **86106797.3**

(22) Date of filing: **20.05.86**

(54) **A multi-needle plate unit with point-saving case for mesotherapeutical use.**

(30) Priority: **21.05.85 IT 1201685**

(43) Date of publication of application:
**30.12.86 Bulletin 86/52**

(45) Publication of the grant of the patent:
**25.07.90 Bulletin 90/30**

(84) Designated Contracting States:
**BE DE FR GB NL**

(56) References cited:
**DE-A-1 491 725**
**DE-A-3 035 009**
**DE-A-3 229 469**
**GB-A-1 096 439**
**US-A-3 595 231**

(73) Proprietor: **Aluigi, Mario**
**Via San Paolo 18**
**I-47040 Corpolo' (Forli) (IT)**
(73) Proprietor: **Mosconi, Paolo**
**Via Dardanelli 17**
**I-47037 Rimini (Prov. of Forli) (IT)**
(73) Proprietor: **Ricciotti, Pier Carlo**
**Via Mentana 36**
**I-47037 Rimini (Prov. of Forli) (IT)**

(72) Inventor: **Aluigi, Mario**
**Via San Paolo 18**
**I-47040 Corpolo' (Forli) (IT)**
Inventor: **Mosconi, Paolo**
**Via Dardanelli 17**
**I-47037 Rimini (Prov. of Forli) (IT)**
Inventor: **Ricciotti, Pier Carlo**
**Via Mentana 36**
**I-47037 Rimini (Prov. of Forli) (IT)**

(74) Representative: **Dr. Ing. A. Racheli & C.**
**Viale San Michele del Carso, 4**
**I-20144 Milano (IT)**

Courier Press, Leamington Spa, England.

## Description

The present invention refers to articles for mesotherapeutical use. "Mesotherapy in this context means a therapeutical method which consists in a localized intradermal injection of very small quantities of pharmaceutical products; the injections are given in the derm or in the superficial hypoderm.

In mesotherapeutical treatments, to better distribute the medical mixtures in the derm thickness, multiple intradermal injections are carried out by using suitable multi-needle heads or plates shaped, in number and placing of the injecting nozzles, according to the specific treatment for which the plate is meant.

Once, the only plates available on the market were the metal ones the high cost of which made their repeated use necessary in spite of the danger of the inevitable accumulation of residual substances in their non-inspectable inner parts.

Presently, instead, disposable plates in plastic material are available, some of which have internal devices meant to make the flow of all the nozzles uniform so as to have a more homogeneous distribution of the medicament in the treated area (US—A—3.595,231).

The use of any known metal or plastic plate presupposes the insertion of the needles on the plate nozzles extending from the side opposite to the one of the collar for insertion of the syringe.

Each injection needle presently on the market comprises at one end a tiny pointed tube and to the other end a plastic cup integral therewith by pressure coupling to a nozzle. Each nedle is placed in a shielding case and further protected in a single envelope. Considering that each plate for mesotherapy is planned to receive an average of seven needles, it can be easily understood how the applying of the needles to a plate can be a long and laborious task.

The aim of the present invention is to obviate to this drawback. A further aim is the improvement of the hygiene of the needles and the plate and their protection from shocks and the protection of the needles after use, the latter operation being presently compulsory, so as to avoid infection.

To reach these aims the subject of this invention is a single-use or disposable plastic plate for mesotherapy characterized in those features according to Claim 1.

According to the claims, various embodiments of point protecting cases are provided.

The new plate has the advantage to considerably reduce the preparation time for the mesotherapeutical treatment, and hence the costs, and to improve the hygienical conditions; further it has the advantage to facilitate, through a single operation (one single case for all the needles), the protection after use, said operation being one of the most dangerous, because of the arrangement and the number of the needles on the plate.

The case, applied before the sterilization treatment, will be removed at the moment of the injection that is after the fixing of the syringe and the expulsion of the air; operations which, thanks to the mentioned case, can be carried out safely because of the impossibility of contamination of the needles from the outside.

Some of the presently preferred embodiments of the invention are described more in detail with reference to the enclosed drawings where:

Figure 1 is a bottom plan view of a disposable circular plate, according to the present invention, without the protective case;

Figure 2 is a front view of the plate of Figure 1;

Figure 3 is a top plan view of the plate of Figure 1;

Figure 4 is a bottom plan view of a disposable linear plate, without the protective case;

Figure 5 is a front view of the plate of Figure 4;

Figure 6 is a top plan view of the plate of Figure 5;

Figure 7 is a right side view of Figure 5;

Figure 8 is a bottom plan view of a first type of case for circular plate;

Figure 9 is a vertical section according to 9—9 of Figure 8;

Figure 10 is a top plan view of the case of Figures 8 and 9;

Figure 11 is a bottom plan view of a type of case similar to the one of Figures 8—10, but for rectangular plates;

Figure 12 is a side partly sectional view of the case of Figure 11;

Figure 13 is a top plan view of the case of Figures 11 and 12;

Figure 14 is a sectional view from 14—14 in Figure 12;

Figure 15 is a bottom plan view of another type of case, for circular plates;

Figure 16 illustrates, partly in side view, partly in vertical section, the case of Figure 15;

Figure 17 is a top plan view of the case of Figures 15 and 16;

Figure 18 is a broken-off front view of a further embodiment of the case according to the present invention;

Figure 19 is a broken-off top view of the case of Figure 18;

Figure 20 is a section according to 20—20 of Figure 18;

Figure 21 is a broken-off top plan view of a further case;

Figure 22 is a left side view of Figure 21, partly broken-off;

Figure 23 is a vertical section according to 23—23 of Figure 21;

Figure 24 is a bottom plan view of a further embodiment of the case;

Figure 25 is a vertical section according to 25—25 of Figure 24;

Figure 26 is a top plan view of the case of Figures 24 and 25;

Figure 27 is a bottom view of another case embodiment, for linear plates;

Figure 28 illustrates, partly in front view, partly in vertical section, the case of Figure 27;

Figure 29 is a plan view of the case of Figures 27 and 28;

Figure 30 is a bottom plan view of another type of case for circular plates;

Figure 31 is a vertical section according to 31—31 of Figure 30;

Figure 32 is a top view of the case of Figures 30 and 31;

Figure 33 is a bottom plan view of a case similar to the one of Figure 30—32, but for linear plates;

Figure 34 illustrates, partly in front view, partly in vertical section, the case of Figure 33;

Figure 35 is a top view of the case of Figure 34;

Figure 36 is a vertical section according to 36—36 of Figure 34;

Figure 37 is a bottom plan view of another type of case, for circular plates;

Figure 38 is a vertical section according to 38—38 of Figure 37;

Figure 39 is a plan view of the case of Figures 37 and 38;

Figure 40 is a bottom plan view of a type of case similar to the one of Figures 37—39, but for rectangular plates;

Figure 41 illustrates, partly in side view, partly in vertical section the case of Figure 40;

Figure 42 is a plan view of the case of Figures 40 and 41;

Figure 43 is a vertical section according to 43—43 of Figure 41;

Figure 44 is a top plan view of a partly open further type of case for circular plates;

Figure 45 is a vertical section according to 45—45 of Figure 44;

Figure 46 illustrates, partly in side view, partly in vertical section, the case of Figure 45;

Figure 47 is a top plan view of a case similar to the one of Figure 44—46, but for rectangular plates;

Figure 48 illustrates, partly in front view and partly in vertical section the case of Figure 47;

Figure 49 is a vertical section according to 49—49 of Figure 48;

Figure 50 is a bottom plan view of a further case embodiment;

Figure 51 illustrates, partly in front view, partly in vertical section, according to 51—51, the case of Figure 50;

Figure 52 is a plan view of the case of Figures 50 and 51;

Figure 53 is a bottom plan view of a further case embodiment, for linear plates;

Figure 54 illustrates, partly in front view and partly in vertical section, the case of Figure 53;

Figure 55 is a plan view of the case of Figures 53 and 54.

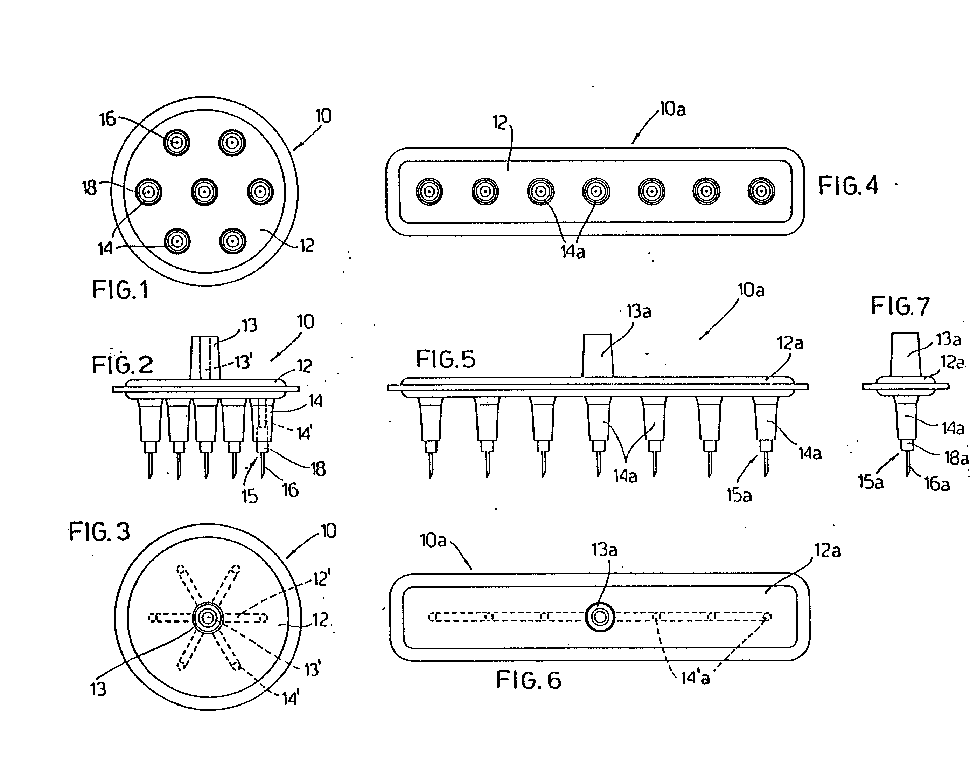

With reference to Figures 1 to 7, they illustrate two different shapes of multi-needle plate unit for mesotherapeutical use, according to the invention. Unit 10 of Figures 1 to 3 has a substantially circular shape in plan view. It comprises a body 12 integral with collar 13 placed on the upper part in Figure 2 and with seven nozzles 14 placed on the bottom part. The collar, which is internally hollow as shown at 13', is used as an attachment for a traditional syringe (not shown). The body 12 of the plate, in a per se known manner, is hollow inside or provided with passages 12' which communicate with internal passages or channels 14' of the nozzles 14 and the internal channel of collar 13, to allow a fluid flow from the latter to the nozzles. The plate, complete of collar and nozzles, is made of plastic material. According to the invention, with each frusto-conical nozzle 14 is integral a needle element 15 which comprises an end-pointed tiny metal tube 16 and a metal sleeve 18 integral with it. Each needle element, or better each sleeve 18, is made integral with the internal passage of the relative nozzle 14 by known means such as glue or adhesive or by moulding the plate directly on the needle elements arranged in a mould. The unit 10 forms thus a unique body, ready for use, and is meant to be disposed of after usage.

The plan shape of the plate can be of any type and the number of nozzles and needles can also vary.

For example, in Figures 4 to 7, a unit 10a of rectangular shape, comprising seven needles in a line, is shown. In said drawings, the same reference numbers of Figure 1 to 3 have been used to indicate similar elements, but with the addition of the 'a' letter. As it appears, also unit 10a forms a whole comprising a plate made up of a body 12a, a collar 13a with a passage 13'a and nozzles 14a with passages 14'a and needle elements 15a.

A case for hygienical protection and safety of the needles of the unit forms part of this invention. This case has a cup or basin form with an edge shaped to abut against the body 12 of the plate which is thus shielded only in the part carrying the needle elements.

The case is generally made of transparent plastic material so as to allow a visual control of the needles particularly during expulsion of the air.

The case can be of various shapes, some of which will be described hereafter with reference to the drawings.

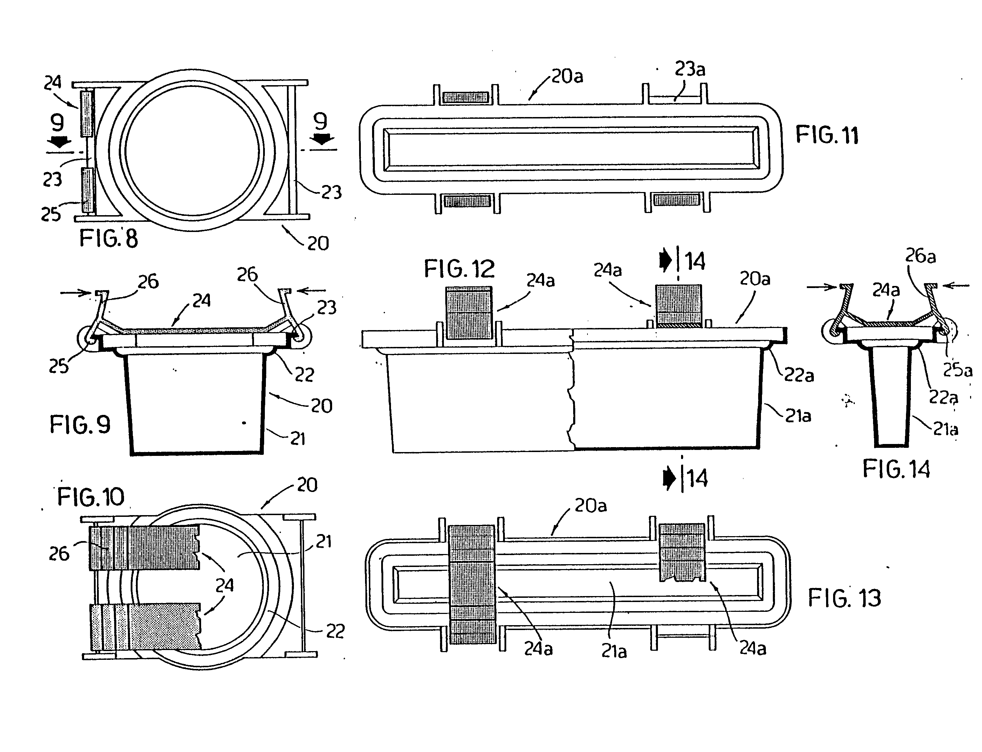

Case 20 of Figures 8 to 10, for circular plates, presents a cup-like part 21 with shaped rim 22 for abutment against the body 12 of a plate 10 and two parallel linear borders or holds 23 where, after insertion of the plate, a couple of elastic band elements 24 fitted with hooks 25 and gripping tabs 26, in a single piece, are hooked to; the subsequent removal of the elements 24 is facilitated by the gripping tabs 26 which when manually pushed towards each other, in the direction of the arrows, cause the disengagement of hooks 25 from the holds 23 below.

Figures 11 to 14 show a case 20a similar to 20, but carried out for linear plates. Case 20a comprises a basin part 21a with edge 22a for abutment against body 21a, rectilinear borders 23a and elastic band elements 24a, fitted with hooks 25a and griping tabs 26a. The elastic elements are disengaged by pushing the tabs according to the arrows of Figure 14, as explained for case 20.

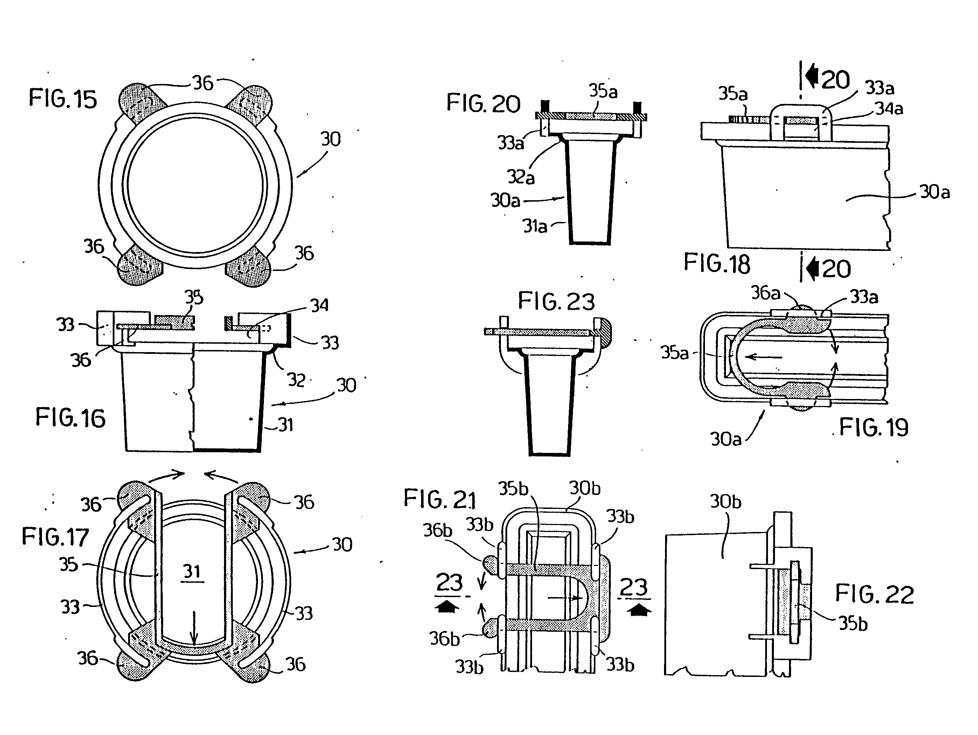

Figures 15, 16 and 17 illustrate a case 30 for circular plates comprising a cup-like part 31 with rim 32 for abutment against a plate, for example, 10, vertical borders 33 provided with horizontal grooves 34 and a flexible fork 35 with tabs 36, generally in number of four. Fork 35, by engaging tabs 36 in grooves 34, locks the case of a plate (not shown). To open the case is sufficient to press the two arms of the fork in the direction of the arrows thus disengaging the tabs from the grooves.

Figures 18, 19 and 20 show a case 30a, similar to 30 but carried out for linear plates. The case 30a comprises a tray or basin part 31a with edge 32 for abutment against a plate 10a, vertical borders 33a provided with horizontal grooves 34a, and flexible forks 35a with opposing tabs 36a, clicked into the grooves. The forks 35a hold the case over the plate. For disengagement, tabs 36a are pressed towards each other in the direction of the arrows.

Figures 21, 22 and 23 show a case 30b, similar to 30a, for linear plates, hold by flexible forks 35b, in this case placed transversely to the longitudinal axis of the case and hooked by means of tabs 36b in suitable holds 33b which jut out from the border of said case.

In this case as well the forks are disengaged with a movement according to the arrows, releasing the case.

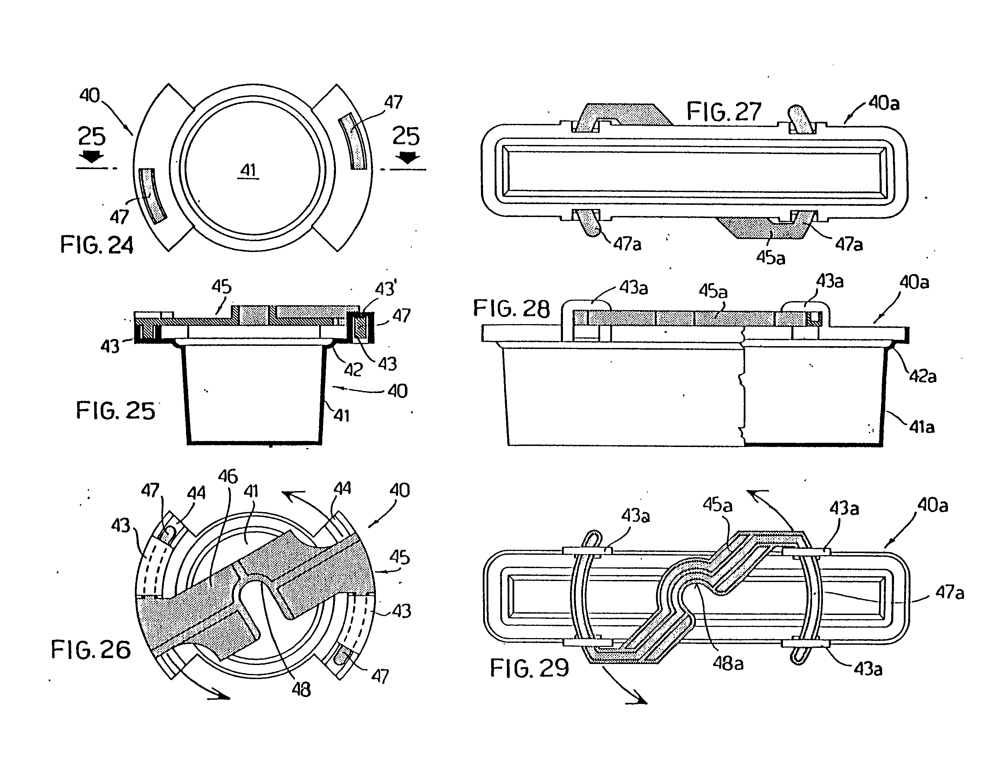

Figures 24, 25 and 26 show a further example of case, indicated by 40, for circular plate. The case 40 comprises a cup-like part 41 with rim 42 shaped like the part of the plate on which it abuts and a vertical border 43 forming two stretches of arc-shaped rails 44 partly covered in 43'. A fixing element is indicated by 45 and comprises a bridging plate 46, and opposing arched tabs 47 which slip inside the holes formed by rims 43, 43'. A housing 48 in the bridging plate receives the upper part of the collar of plate 10 when the case is fitted over it. To remove the case, it is sufficient to rotate the fixing element 45 around said collar until tabs 47 slip out from the holes provided in the upper rim 43 of the case.

Figures 27, 28 and 29 show a case 40a similar to case 40 of Figure 24 to 26, but carried out for linear plates. Case 40a comprises a cup or basin part 41a, an edge 42a from which eyelets 43a extend so to engage a bridging fixing element 45a fitted with tabs 47a. The disengagement of element 45 is obtained by rotating around the axis of the collar 13a of a plate 10a, in the direction of the arrows of Figure 29.

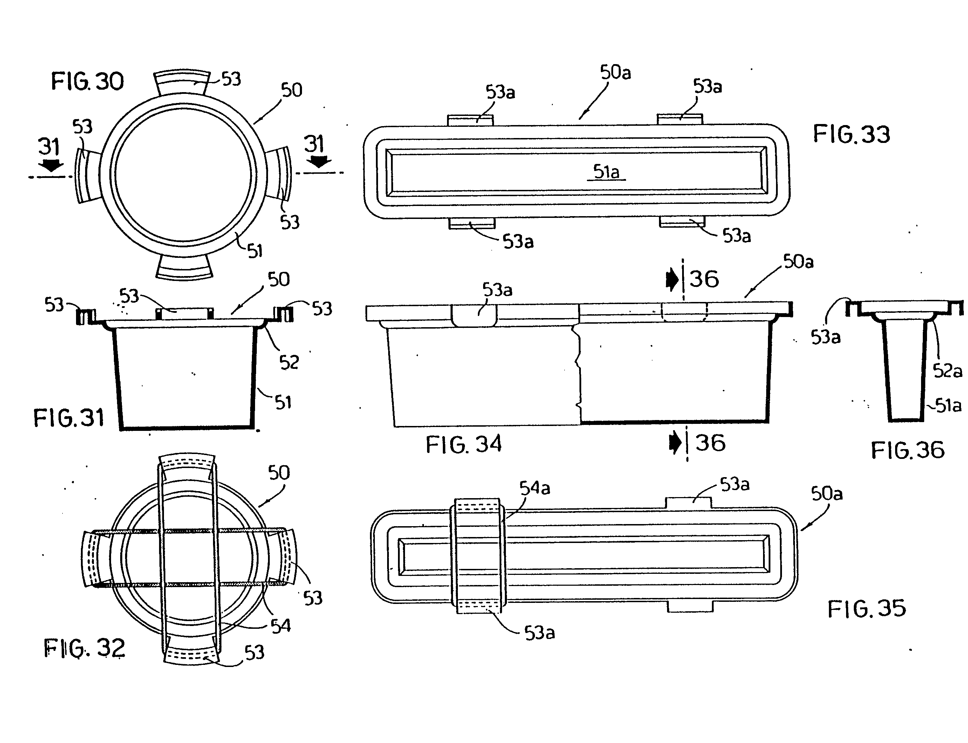

Figures 30, 31 and 32 show a further case embodiment, indicated by 50, for circular plates 10. Case 50 comprises as usual a basin part 51 to lodge and protect needles and nozzles of the plate; a rim 52 for abutment of the plate and four radial extensions ending in a hook 53, placed crosswise. The case is kept to the plate by means of two elastic bands 54 and can be easily removed by removing the bands.

Figures 33 to 36 show a case 50a similar to 50 but for linear plates. The case 50a comprises a basin part 51a of rectangular shape, with edge 52a. In this case the opposing hooks 53a are placed on the longer sides of the rectangle and linked two by two by means of elastic bands 54a.

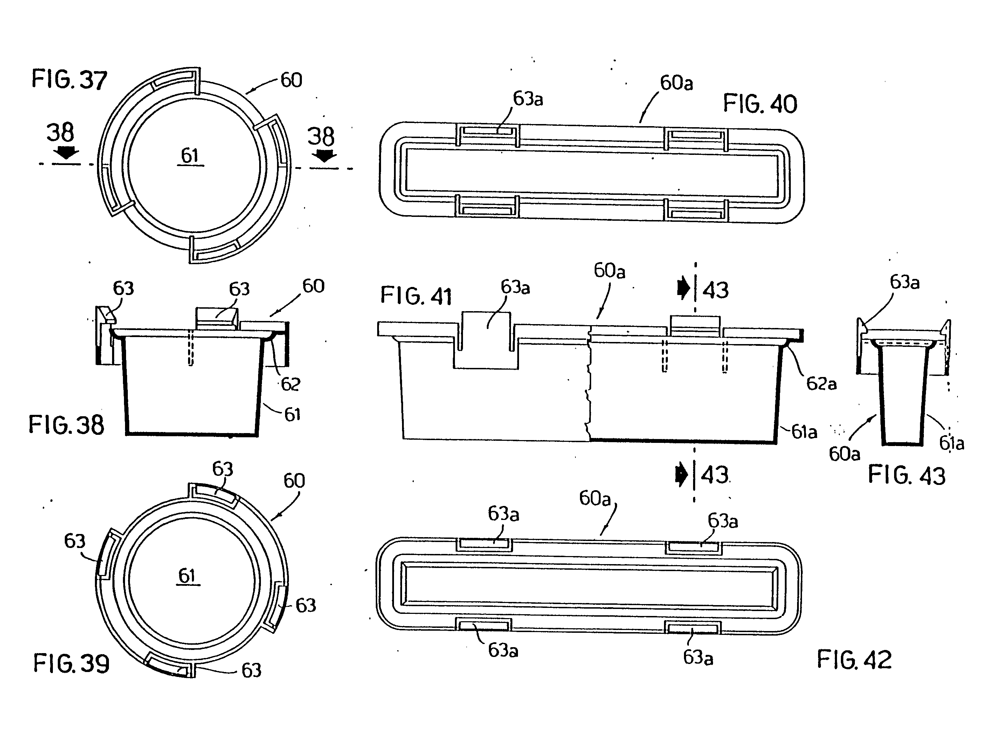

Figures 37, 38 and 39 show a further case embodiment for circular plates, indicated by 60. It comprises a basin part 61 to lodge the needles and nozzles of the plate, with abutting rim 62 from which resilient hooks or teeth 63 protrude, defined on the sides by slits; the temporary resilient yielding of the teeth allow them to automatically grip the external rim of the plate, pressed on insertion. To free the plate it is sufficient to spread apart the resilient teeth.

Figures 40 to 43 show a case 60a for linear plates, similar to case 60; it comprises a basin 61 with edge 62. The resilient teeth 63a protruding from the edge are placed two by two along the longitudinal sides.

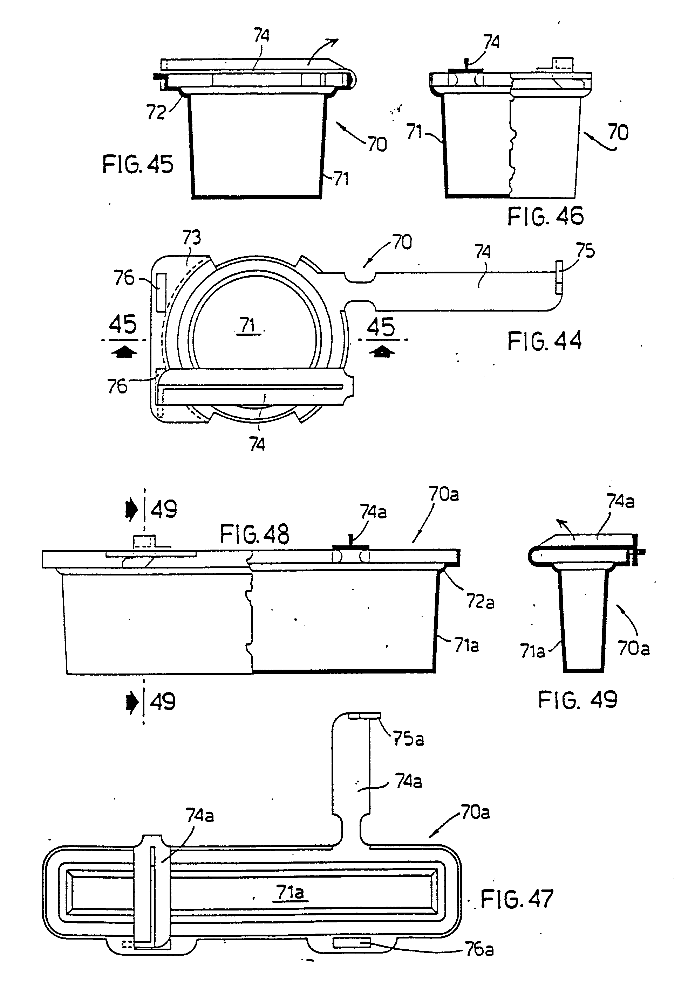

Figures 44, 45 and 46 show a further case embodiment, indicated by 70, for circular plates. The case comprises a basin part 71 with rim 72, an enlarged part 73 to which two flexible possibly ribbed strips 74 are integral with, fitted at the end with tooth 75 which engages in eyelet 76. It should be noted that all the cases described for circular plates leave the plate free on two opposite edging borders to allow gripping for connection by rotation to a syringe.

Figures 47, 48 and 49 show the version of case 70 for linear plates. The rectangular case is indicated by 70a; number 71a indicates the basin and 72a the rim. From the longer sides, two flexible, possibly ribbed, strips 74a extend, the end of which engages in eyelets 76a.

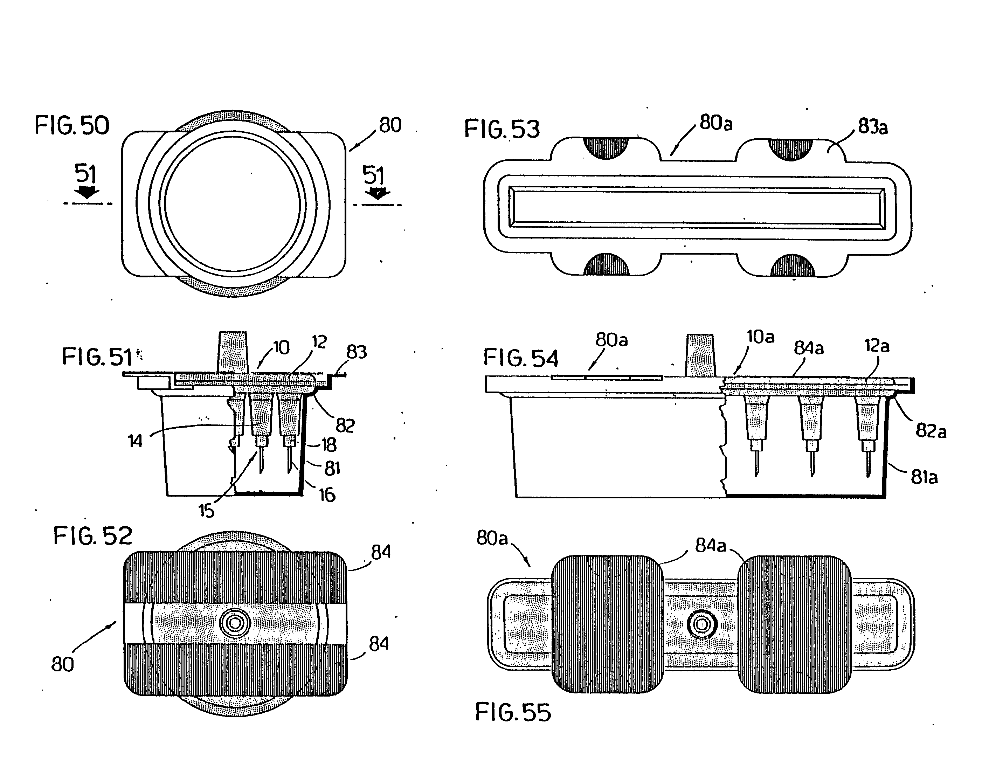

Finally figures 50, 51 and 52 show a case 80 for circular plate. Case 80 presents a basin part 81 with abutting rim 82, an enlarged part 83 and is fixed by two strips 84 of a material suitable for gluing or welding, on the horizontal surfaces of the case enlarged part. Figure 51 show a plate 10 lodged in the case.

Figures 53, 54 and 55 show a case for a linear plate; Figure 54 show a disposable linear plate lodged in it. The case comprises a basin part 81a with an edge 82a an enlarged part 83a and it is fixed, like for the previous embodiment, by two strips 84a made of a material suitable to be glued or welded to the edge of the case.

In conclusion of what has been explained, attention should be drawn to the fact that both the disposable plates and the relating point-saving cases, still without leaving the field of the requested patent, are susceptible of formal and substantial modifications determined by work necessities or, more simply, by the number and the placing of the injecting needles required.

## Claims

1. A multi-needle plate unit for mesotherapeutical use, comprising a body (12, 12a), a collar (13, 13a) extending from the body, for attachment to a syringe; a plurality of nozzles (14, 14a) extending from the side of the body opposite to the collar; a needle element for each nozzle and in communication with the collar, characterized in that each needle is integral with the relevant nozzle and in that the unit further comprises a case (20, 20a; 30, 30a...80, 80a) made of plastic material, comprising a cup or basin part (21, 21a...81, 81a) which lodges all the needles, a rim (22, 22a...82, 82a) for abutment against the body and removable holding elements (24...84) to keep the case on the body.

2. A unit according to Claim 1, each needle element comprising a tiny pointed tube and an integral metallic sleeve, characterized in that each sleeve (18, 18a) is made integral with the relevant nozzle.

3. A unit according to Claim 1, characterized in that each needle element (15, 15a) is made integral with the relevant nozzle (14, 14a) during the moulding of the plate.

4. A unit according to Claim 1, characterized in that each needle element (15, 15a) is made integral with the relevant nozzle (14, 14a) by means of adhesives.

5. A unit according to Claim 1, for circular plate or body, characterized in that the plate fitted with the case has two opposite clear sides of the case, so that the plate can be gripped between two fingers and in two diametrically opposite positions during insertion of the syringe.

6. A unit according to Claim 1, characterized in that the case (20, 20a) has a jutting rim (23, 23a) and the holding elements are formed by elements (24, 24a) in a relatively resilient material, fitted with hooks (25, 25a) apt to be engaged on said jutting edges and with gripping tabs (26, 26a).

7. A unit according to Claim 1, characterized in that the case (30, 30a) has an axially extended rim, fitted with slits or grooves (34, 34a, 34b) and the holding elements comprise a resilient fork (35, 35a, 35b) with tabs (36, 36a, 36b) which engage in the grooves.

8. A unit according to Claim 1, characterized in that the case comprises a rim (43, 43a) with eyelets (43', 43'a) and the holding elements (40, 40a) is formed by arched tabs (47, 47a) and a lodging (48, 48a) apt to receive the collar of a plate and so rotate around it.

9. A unit according to Claim 1, characterized in that the case (50; 50a) has opposing hooking parts (52, 52a) protruding from the edge and the holding elements are elastic bands (54, 54a).

10. A unit according to Claim 1, characterized in that the case (60, 60a) comprises resilient teeth (63, 63a) protruding from the edge, which engage in the plate.

11. A unit according to Claim 1, characterized in that the case comprises two flexible strips (74, 74a) each strip extending from the case upper edge and having a hooking tooth (75, 75a), and eyelets (76, 76a) on the opposite edge.

12. A unit according to Claim 1, characterized in that the holding elements are formed by strips (84, 84a) of material glued or at any rate made adhesive to a surface integral with the basin of the case.

**Patentansprüche**

1. Vielfachnadelplatteneinheit für den mesotherapeutischen Gebrauch mit einem Körper (12, 12a), einem aus dem Körper stehenden Bund (13, 13a) zum Anbringen einer Spritze, einer Vielzahl von aus dem Körper auf der dem Bund gegenüberliegenden Seite sich erstreckenden Düsen (14, 14a), mit einem mit dem Bund in Verbindung stehenden Nadelelement für jede Düse, dadurch gekennzeichnet, daß jede Nadel an der entsprechenden Düse befestigt ist und daß die Einheit außerdem ein Kunststoffgehäuse (20, 20a; 30, 30a usw., 80, 80a) umfaßt, das eine Schale (21, 21a..., 81, 81a) zur Aufnahme aller Nadeln aufweist und einen sich auf den Körper abstützenden Rand (22, 22a..., 82, 82a) und lösbare Halteelemente (24...84) zum Festhalten des Gehäuses auf dem Körper hat.

2. Einheit nach Anspruch 1, bei der jedes Nadelelemente ein spitzes Röhrchen und eine feste Metallmuffe aufweist, dadurch gekennzeichnet, daß jede Muffe (18, 18a) an der entsprechenden Düse fest angebracht ist.

3. Einheit nach Anspruch 1, dadurch gekennzeichnet, daß jedes Nadelelement (15, 15a) an der entsprechenden Düse (14, 14a) während des Spritzgiessens der Platte fest angebracht wird.

4. Einheit nach Anspruch 1, dadurch gekennzeichnet, daß jedes Nadelelement (15, 15a) an der entsprechenden Düse (14, 14a) mit Klebemitteln fest angebracht ist.

5. Einheit nach Anspruch 1, für Platte oder runden Körper, dadurch gekennzeichnet, daß die mit dem Gehäuse vervollständigte Platte zwei gegenüberliegende nicht vom Gehäuse bedeckte Ränder aufweist, damit man die Platte während des Einsteckens der Spritze mit zwei Fingern in diametral gegenüberliegenden Bereichen festspannen kann.

6. Einheit nach Anspruch 1, dadurch gekennzeichnet, daß das Gehäuse (20, 20a) einen vorstehenden Rand (23, 23a) aufweist und die Halteelemente aus Elementen (24, 24a) aus verhältnismäßig elastischem Material gebildet, mit Haken (25, 25a) zum Eingriff in die vorstehenden Ränder und mit Handgriffen (26, 26a) versehen sind.

7. Einheit nach Anspruch 1, dadurch gekennzeichnet, daß das Gehäuse (30, 30a) einen axial verlaufenden Rand aufweist, der mit Fenster oder Nuten (34, 34a, 34b) versehen ist und die Halteelemente ein elastische Gabel (35, 35a, 35b) mit Zungen (36, 36a, 36b) zum Eingriff in die Nuten aufweisen.

8. Einheit nach Anspruch 1, dadurch gekennzeichnet, daß das Gehäuse einen Rand (43, 43a) mit Ösen (43', 43'a) aufweist und das Halteelement (40, 40a) bogenförmige Zungen (47, 47a) und einen Sitz (48, 48a) zur Aufnahme des Bundes einer Platte aufweist, um sich darin drehen zu können.

9. Einheit nach Anspruch 1, dadurch gekennzeichnet, daß das Gehäuse (50, 50a) sich vom Rand erstreckende gegenüberliegende Hakenteile (52, 52a) aufweist und die Halteelemente elastische Streifen (54, 54a) sind.

10. Einheit nach Anspruch 1, dadurch gekennzeichnet, daß das Gehäuse (60, 60a) vom Rand ausgehende elastische Zähne (63, 63a) aufweist, die in die Platte eingreifen.

11. Einheit nach Anspruch 1, dadurch gekennzeichnet, daß das Gehäuse zwei beigsame Streifen (74, 74a) umfaßt, wobei sich jeder Streifen vom oberen Gehäuserand erstreckt und Einhakzähne (75, 75a) und Ösen (76, 76a) auf dem gegenüberliegenden Rand aufweist.

12. Einheit nach Anspruch 1, dadurch gekennzeichnet, daß die Halteelemente aus Bändern (84, 84a) aus klebbarem oder in irgend einer Weise auf einer festen Oberfläche an der Schale des Gehäuses haftbarem Material bestehen.

**Revendications**

1. Unité de plaque à aiguilles multiples utilisée en mésothérapie, comprenant un corps (12, 12a), un petit collier (13, 13a) s'étendant hors du corps, pour y fixer une seringue; une multiplicité de buses (14, 14a) s'étendant hors du corps, du côté du corps opposé au petit collier; un élément à aiguille pour chaque buse et en communication avec le petit collier, caractérisée en ce que chaque aiguille est solidaire de la buse correspondante et que l'unité comprend en outre un boîtier en matière plastique (20, 20a; 30, 30a, etc. 80, 80a) comprenant une partie en cuvette (21, 21a,...81, 81a) qui abrite toutes les aiguilles, un bord (22, 22a...82, 82a) servant d'appui contre le corps, et des éléments de retenue (24...84) amovibles pour retenir le boîtier sur le corps.

2. Unité selon la revendication 1, chacun des éléments à aiguille comprenant un petit tube pointu et un manchon métallique solidaire, caractérisée en ce que chaque manchon (18, 18a) est rendu solidaire de la buse correspondante.

3. Unité selon la revendication 1, caractérisée en ce que chacun des éléments à aiguille (15, 15a) est rendu solidaire de la buse correspondante (14, 14a) au cours de la formation en moulage de la plaque.

4. Unité selon la revendication 1, caractérisée en ce que chacun des éléments à aiguille (15, 15a) est rendu solidaire de la buse correspondante (14, 14a) à l'aide de colles.

5. Unité selon la revendication 1, pour plaque ou corps circulaire, caractérisée en ce que la plaque munie de boîtier présente deux bords opposés découverts par le boîtier, pour pouvoir serrer, à l'aide de deux doigts et dans des zones diamétralement opposées, la plaque au moment de l'introduction de la seringue.

6. Unité selon la revendication 1, caractérisée en ce que le boîtier (20, 20a) présente un bord saillant (23, 23a) et que les éléments de retenue se constituent d'éléments (24, 24a) en matériau relativement élastique, munis de crochets (25, 25a) pour l'application sur lesdits bords saillants et d'ailettes de prise (26, 26a).

7. Unité selon la revendication 1, caractérisée en ce que le boîtier (30, 30a) présente un bord étendu axialement, muni de fenêtres ou cannelures (34, 34a, 34b) et les éléments de retenue comprennent une fourche élastique avec des languettes (36, 36a, 36b) à introduire dans les cannelures.

8. Unité selon la revendication 1, caractérisée en ce que le boîtier comprend un bord (43, 43a) avec des oeillets (43', 43'a) et l'élément de retenue (40, 40a) comprenant des languettes à arc (47, 47a) et un siège (48, 48a) permettant d'abriter le petit collier d'une plaque et de pouvoir tourner autour de celui-ci.

9. Unité selon la revendication 1, caractérisée en ce que le boîtier (50, 50a) présente des parties à crochet opposées (52, 52a) s'étendant hors du bord et les éléments de retenue sont des bandes élastiques (54, 54a).

10. Unité selon la revendication 1, caractérisée en ce que le boîtier (60, 60a) comprend des dents élastiques (63, 63a) s'étendant hors du bord, qui retiennent la plaque.

11. Unité selon la revendication 1, caractérisée en ce que le boîtier comprend deux bandes flexibles (74, 74a), chacune s'étendant hors du bord supérieur de cette dernière et présentant une dent d'accrochement (75, 75a) et des boutonnières (76, 76a) sur le bord opposé.

12. Unité selon la revendication 1, caractérisée en ce que les éléments de retenue se constituent de centures (84, 84a) en matériau collé ou de toute façon rendu adhérent à une surface solidaire de la cuvette du boîtier.

FIG.1

FIG.2

FIG.3

FIG.4

FIG.5

FIG.6

FIG.7

FIG. 8

FIG. 9

FIG. 10

FIG. 11

FIG. 12

FIG. 13

FIG. 14

EP 0 205 915 B1

FIG.15

36

30

36

36

36

FIG.16

35

34

33

33

36

32

30

31

FIG.17

36

36

35

31

33

33

36

36

30

FIG. 20

35a

33a

33a

32a

30a

31a

35a

33a

34a

30a

FIG.18

20

20

FIG. 23

FIG. 19

36a

33a

35a

30a

FIG. 21

35b

30b

36b

33b

33b

23

23

36b

33b

33b

30b

FIG. 22

35b

EP 0 205 915 B1

FIG. 24

FIG. 25

FIG. 26

FIG. 27

FIG. 28

FIG. 29

EP 0 205 915 B1

FIG. 30

53 — 50

53

31 — 31

53

51

**FIG.31**

53 — 53 — 50

53 — 53

52

51

**FIG.32**

50

53

54

53

50a

53a — 53a

51a

**FIG.33**

53a — 53a

53a — 36 — 50a

53a

**FIG. 34** — 36

53a — 52a

51a

**FIG.36**

54a — 53a — 50a

53a

**FIG. 35**

EP 0 205 915 B1

FIG. 37 FIG. 38 FIG. 39 FIG. 40 FIG. 41 FIG. 42 FIG. 43

FIG. 45

74

72

70

71

FIG. 46

74

71

70

FIG. 44

73

76

45

71

45

76

74

70

74

75

FIG. 48

49

74a

70a

72a

71a

49

FIG. 49

74a

71a

70a

75a

74a

70a

74a

71a

FIG. 47

76a

FIG.53

FIG.54

FIG.55

FIG.50

FIG.51

FIG.52